# EUROPEAN PATENT APPLICATION

(11) **EP 2 072 110 A1**
(43) Date of publication of application: **24.06.2009**
(21) Application number: 08022028.8
(22) Date of filing: 18.12.2008
(51) Int. Cl.: B01D 53/38, B01D 53/78, A61L 9/01

(54) **Method for removal of waste gasses from air**

(30) Priority: 18.12.2007 NL 1034836
(71) Applicant: ARN BV, 6551 DZ Weurt (NL)
(72) Inventor: Drewes, Peter, 6711 JV Ede (NL); Van Gorkum, Gerardus Henricus Johannes Paulusulus, 6562 ML Groesbeek (NL); Rozestraten, Arnoldus Franciscus Johannes, 6533 WR Nijmegen (NL); Van Winden, Antonius Adrianus Franciscus, 6566 XP Millingen Aan den Rijn (NL)
(74) Representative: Van Someren, Petronella F. H. M.

(57) **Abstract**

The present invention relates to a method for removing waste gases from air deriving from composting waste, comprising of first making the air very moist by spraying alkaline water into the air and subsequently mixing peroxide and ozone into the airflow. The present invention further relates to a method wherein the air comprises between 20 mg and 10000 mg of carbon per m³ air, preferably between 50 mg and 1000 mg of carbon per m³ air. The present invention also relates to a method wherein the air has a temperature between 20°C and 80°C, preferably between 40°C and 70°C, when first made very moist.

## Description

The present invention relates to a method for removal of waste gases from air deriving from composting waste.

The present waste treatment industry is characterized by the need to process large quantities of mainly domestic waste, wherein the waste must be processed in inexpensive, economic manner. It is important here that released fractions, both the solid fraction and the gaseous fraction, are of a quality such that such fractions can be returned to the environment. On the one hand the waste flows and waste fractions and gaseous emissions change constantly due to the multiformity, variety and variability of the presented waste. On the other hand the current regulations are frequently amended, which amounts mainly to readjustment, such as for instance adjusting emission norms downward, whereby industry is compelled to modify waste treatment processes in order to be able to cope with these changing situations.

In regular waste treatment, which takes place on the basis of mainly mechanical and thermal processes, prior to incineration the presented waste is first broken up and then separated into an organic, readily combustible fraction and a poorly combustible fraction. The readily combustible fraction comprises dry components such as paper, synthetic materials and plastics. The poorly combustible fraction, or sieve fraction, created here comprises wet organic components. The quantity of sieve fraction is not inconsiderable; about 400 kilos of sieve fraction is in this way created for each tonne of domestic waste for processing. This incombustible fraction or sieve fraction must be dumped, and this entails high cost.

An alternative method for processing domestic waste relates to a method wherein the waste is pretreated by means of microbiological activity for the purpose of further waste treatment. Despite waste being separated substantially at source, the waste for processing comprises suitable quantities of organic material to support such a microbiological composting reaction. The organic material which can serve here as substrate for such a biological degrading process originates from food remnants, green waste, garden waste, coffee grounds, eggshells, paper and the like.

In order to enable such a biological degrading process or composting process as pretreatment, the waste for processing is placed into a space to be conditioned, such as a drying drum or one or more tunnels. Continuous aeration takes place herein, and the waste is kept in motion so that the biological degrading processes, which involve micro-organisms such as bacteria, but also fungi or yeasts, can take place. During this degradation, which can take place substantially without the addition of heat, heat is generated by means of micro-organisms, whereby the temperature in this system can vary between ambient temperature at the start of the degrading process and can rise to between 60°C and 80°C as the degradation proceeds. A large part of the moisture present in the waste will hereby evaporate, whereby the favourable effect occurs that the quantity of waste for further processing decreases significantly and the waste for further treatment becomes drier. Once the biological degrading process has progressed sufficiently, there remains a solid fraction. This fraction can optionally be incinerated after the removal of iron.

The fraction with waste gases is obtained by continuous aeration and extraction during the biological degrading process. Such a fraction will then mainly comprise volatile degradation products generated by micro-organisms. As a result of the combination of continuous movement and extraction, solid organic matter can to a limited extent also enter the gaseous waste flow.

The substantially gaseous fraction with waste gases must be further processed or cleaned because this fraction comprises waste gases harmful to the environment.

The gaseous waste fraction consists mainly of gaseous carbon-containing compounds such as aromatics, aliphatics, fatty acids, ketones, aldehydes and the like and, to a more limited extent, solid organic matter. The totality of these compounds is referred to here as Total Organic Carbon (TOC). The volatile carbon-containing compounds in the TOC are referred to here as Volatile organic Carbon (VOC).

For the purpose of processing such a gaseous waste flow use can be made of technologies based on biological filters, such as on the basis of processed wood chippings and/or bark, or thermal post-combustion.

The characteristic of the use of biological filters is that such a technology is suitable when the waste gases for treatment have a relatively low concentration of VOC, such as 1 g/m³ and lower, and have a temperature of a maximum of 35-40°C. At temperatures of the air with waste gases for treatment which lie above 35-40°C the air must therefore be cooled before the actual treatment can take place. Another drawback occurring in this technology is that the biological filter cannot process large quantities of water vapour. This technology is further characterized in that there is a narrow temperature bandwidth at which the technology works well. Temperature fluctuations of more than 2°C have a negative effect on the treatment capacity. In addition, the treatment capacity of such a filter is relatively low, whereby such a biological filter takes up a lot of space in order to have a suitable treatment capacity.

In the case of waste gases which have a relatively high temperature, in the range of 40°C and above, and in which the concentrations of VOC are relatively high, at 1 g/m³ and higher, a technology based on thermal post-combustion is suitable for removal of waste gases. The drawback occurs here that this technology is based on the additional supply of heat (or energy), which makes this technology expensive and less cost-effective. The combustion of fossil fuels also involves a high level of CO₂ emission.

It is therefore an object of the present invention to provide a method for removing waste gases from air deriving from composting waste, wherein the above stated drawbacks occurring during the removal of waste gases are obviated.

In the research leading to the present invention it was ascertained that, surprisingly, it is possible to degrade waste gases deriving from composting waste into substantially CO₂ and H₂O by utilizing chemical radicals and specific oxidation reactions and specific methodologies for causing optimal degradation of waste gases into mainly CO₂ and H₂O.

In order to achieve the above stated object the present invention provides a method for removing waste gases from air deriving from composting waste, comprising of first making the air very moist by spraying alkaline water into the air and subsequently mixing peroxide and ozone into the airflow.

The method according to the invention can be applied to many types of waste gases from air deriving from composting waste. Such waste preferably comprises domestic waste, but also compost waste or other organic waste. This waste can be treated in various devices for processing of such waste, such as an Integral Biological Treatment (IBT) installation, a fermentation installation, composting installation, biological waste treatment installation or biological drying installation. Characteristic for the method according to the present invention is that the air from such devices derives from composting.

'Composting' is understood here in the broadest sense of the word. Composting consists of an optionally controlled fermenting process, in which organic materials such as organic waste, green waste, organic domestic waste, food remnants, kitchen waste and so on are converted into compost (humus) in the presence of oxygen. Mainly released during this process are heat, carbon dioxide and water. The released heat contributes here toward the evaporation of the present and released water. This composting can also take place in a mixture of organic components and non-organic components (such as plastics, synthetic materials, rubble, leather, textile), wherein the full or partial conversion of the organic components present by micro-organisms produces the heat.

Waste gases from air deriving from composting waste are here understood to mean the substantially gaseous fraction with waste gases consisting of TOC and VOC which are released during composting of organic waste, green waste, organic domestic waste, food remnants, kitchen waste and the like. This composting can take place here in an installation adapted for this purpose, such as an Integral Biological Treatment (IBT) installation, a fermentation installation, composting installation, biological waste treatment installation, biological drying installation and the like. TOC and VOC are defined here in accordance with the current European NEN-ISO standard.

The method according to the present invention has the further advantage that, for air deriving from composting waste, use need only be made of chemistry on the basis of alkaline water, peroxide and ozone. An effect which occurs here is that the use of alkaline water, ozone and hydrogen peroxide does not contribute toward contamination of the alkaline water with which the air is made very moist. The alkaline water hereby comprises no or minimal amounts of impurities which can for instance occur during mineralization. This alkaline water can also be easily recovered, substantially without applying purification, for direct reuse as alkaline water. It will also be possible to discharge or drain the alkaline water in simple manner due to the degree of absence of impurities, or the quality of the alkaline water.

Another advantage of the present method is that, by first making the air with waste gases very moist, the substantially poorly soluble VOC and/or TOC are brought into contact with ozone and hydrogen peroxide in suitable manner by means of reactive, very small droplets. The very small reactive droplets provide a very large reaction area, so that the VOC and/or TOC can react in appropriate and efficient manner with ozone and hydrogen peroxide. The full or partial degradation reaction of VOC and/or TOC to ultimately CO₂ and H₂O will hereby proceed in suitable manner. The large reaction area is especially important in the case of air with waste gases from composting waste, since the quantities of TOC and/or VOC herein are relatively low. By increasing the contact area the TOC and/or VOC can react efficiently in appropriate manner.

A surprising effect observed during the research leading to the invention is that a synergistic effect occurs when ozone and hydrogen peroxide are simultaneously added according to the invention during the removal of waste gases from a gas, in this case air deriving from composting waste, wherein the gas has first been made very moist with alkaline water. Table I shows that a TOC reduction of 15% takes place when only hydrogen peroxide is added, a reduction of 50% takes place when only ozone is added, and a reduction of up to 89% takes place when ozone and hydrogen peroxide are administered simultaneously. Such an effect could be seen as very surprising since this is a method in which a gas is treated with ozone and hydrogen peroxide in order to remove waste gases from such a gas.

**Table 1: TOC reduction through treatment of air from composting waste.**

| Treatment | TOC in (mg/m³) | TOC out (mg/m³) | TOC reduction |
|---|---|---|---|
| H₂O₂ | 123.6 | 104.0 | 16% |
| H₂O₂ | 147.6 | 125.0 | 15% |
| O₃ | 120.9 | 62.0 | 49% |
| O₃ | 123.6 | 61.1 | 51% |
| H₂O₂+O₃ | 114.3 | 17.7 | 85% |
| H₂O₂+O₃ | 101.0 | 10.7 | 89% |

An aspect of the invention relates to the alkaline water. Alkaline water is here understood to mean an aqueous solution to which any suitable base is added, whereby the pH of the water lies in the alkaline range and whereby the method according to the invention proceeds in suitable manner. A pH of the alkaline water of at least 8 is required here. Through the use of alkaline water an alkaline environment is provided wherein the ozone and hydrogen peroxide can react in appropriate manner with the TOC and/or VOC from the air deriving from composting waste.

Both the added hydrogen peroxide and the ozone were administered in excess in all treatments as stated in table 1.

Another advantage of the method according to the invention is that the amount of odour has decreased dramatically. The number of odour units, expressed in GE/m³, was found to have decreased by 97%. These figures are shown in table II.

**Table II: Odour unit reduction through treatment of air from composting waste.**

| Odour unit (GE/m³) | Before peroxide/ozone | After peroxide/ozone | Efficiency (%) |
|---|---|---|---|
| Measurement 1 | 17,232 | 300 | 97 |
| Measurement 2 | 38,164 | 1,078 | 97 |

One odour unit is defined here as the odour concentration at which, of a group of people with an average sense of smell, half can still distinguish the odour from odour-free air. It will hereby be apparent to the skilled person what is understood by odour unit.

The method of mixing ozone and/or hydrogen peroxide in the airflow can take place in a number of ways. Ozone can for instance also be supplied to alkaline water, after which it can be mixed into the airflow. Hydrogen peroxide can also be supplied to alkaline water, after which it can be mixed into the airflow. Both ozone and hydrogen peroxide can be supplied to the alkaline water, after which this water can be mixed into the airflow. Any method of mixing ozone and hydrogen peroxide into the airflow would in fact work. In this respect it is possible to envisage injectors, ejectors, compressors, venturis and the like. It is important that the alkaline water with ozone and/or hydrogen peroxide is introduced into the airflow by providing the water in droplet form. Droplet sizes of between 20 and 300 µ, in particular droplet sizes of 50-100 µ, are desirable here. At these droplet sizes an increase in the contact area occurs, thereby resulting in a high reaction capacity of the alkaline water droplets, ozone, hydrogen peroxide and the VOC and/or TOC. The chance of a reaction hereby occurring between ozone, hydrogen peroxide and the VOC and/or TOC is hereby also increased. Provision is also made here that ozone and/or hydrogen peroxide can be mixed into the airflow by means of a plurality of feeds or feed points. It is the case here that droplet distributors, atomizers, sprayers and/or multipoint injectors can be used. The level of power consumption required to obtain droplets of said sizes can hereby be described as favourable or low.

Another advantage of the present invention is that no additional energy, for instance in the form of combustion, need be supplied in order to remove the TOC and/or VOC in suitable manner. The method according to the present invention is also highly suitable for processing large quantities of water vapour, wherein the removal of waste gases according to the invention is not substantially reduced or impeded by the water vapour (or a relatively high humidity). The device required for a method according to the present invention also takes up relatively little space, whereby the technology is advantageous in establishments where little space is available.

Another aspect of the invention relates to the air deriving from composting waste which comprises between 20 mg and 10000 mg of carbon per m³ air, preferably between 50 mg and 1000 mg of carbon per m³ air. The quantity of TOC in the air is determined here by establishing the amount of carbon as determined in accordance with the current European NEN-ISO standard. It will hereby be apparent to the skilled person that the quantity of TOC can be expressed as the quantity of carbon. A measurement where a quantity of 150 mg C/m³ of air is determined is thus understood here to mean 150 mg of TOC.

An advantage of the present invention is that the provided method has a high degree of flexibility and (over)capacity for removing varying quantities of carbon from the supplied air. Waste gases from air deriving from composting waste are characterized in that they are particularly variable in respect of the temperature of the waste flow and the quantities or concentrations of TOC and/or VOC. The quantities of carbon in the air to be removed can thus be anticipated by means of the method according to the invention, while the removal of carbon takes place in appropriate manner. It has even been found that this flexibility can be further optimized by taking further specific measures such that the method according to the invention can be modified to many specific circumstances and conditions under which the air with waste gases to be supplied can be processed.

Another aspect of the invention is the air, which has a temperature between 20°C and 80°C, preferably between 40°C and 70°C, when first made very moist. Yet another aspect of the invention is that the air has a temperature between 20°C and 80°C, preferably between 40°C and 70°C, during the spraying of alkaline water. Yet another aspect of the invention is that the airflow has a temperature between 20°C and 80°C, preferably between 40°C and 70°C, during the mixing of peroxide and ozone.

According to the present method of the invention, the temperature of the air for treatment can fall within the broad range as indicated above, at which the method continues to operate well. An advantage here is therefore that in the method according to the present invention the temperature of the supplied air requires no adjustment, or does so to a lesser extent, to the process of removing waste gases from the air. This advantage applies to the air which is first made very moist and to the temperature of the air when alkaline water is sprayed, as well as to the temperature of the airflow when peroxide and ozone are mixed. The necessity of cooling the supplied air is particularly reduced or even absent in the present invention. An increase in the temperature of the air for treatment does not occur either, or to a lesser extent. The air from a device where composting takes place can hereby be treated according to the invention without or with minimal changes in temperature.

A further advantage of the invention is that the temperature of the air for treatment can display rather great fluctuations, i.e. need not have a very constant temperature. Even in the case of sudden, rapid or great temperature fluctuations the removal of waste gases continues in suitable manner.

Other technologies are for instance characterized in that they have a narrower temperature bandwidth within which the air for treatment must fall, or that these methods do not operate optimally in the case of great temperature fluctuations. In the present method adjustment of the temperature of the air for treatment is not necessary, or only to a very limited extent.

It is a further advantage of the present invention that the air for treatment can comprise larger quantities of water vapour at higher temperatures, wherein the removal of waste gases according to the invention continues. This is because the waste for processing is characterized in that it comprises large quantities of water or that large quantities of water are released during the composting of waste. The removal of waste gases from this air continues efficiently in the case of large quantities of water vapour. The water vapour-processing capacity and flexibility of the method has been found to be highly advantageous here. Although the method according to the invention can process air for treatment with a high air humidity, first making the air very moist is necessary in order to create an alkaline environment thereby the removal of waste gases proceeds in suitable manner.

In an embodiment of the invention the ozone is provided in a quantity of between 0.05 and 1.70 grams of ozone, preferably between 0.20 and 0.70 grams of ozone, per gram of carbon in the air deriving from composting waste.

In another embodiment the hydrogen peroxide is provided in a quantity of between 0.5 and 35 grams of hydrogen peroxide of 35% by weight, preferably between 0.1 and 3.5 grams of hydrogen peroxide of 35% by weight, per gram of carbon in the air deriving from composting waste.

At said quantities of ozone and hydrogen peroxide according to such embodiments these substances are provided in a suitable ratio, mix and quantity in order to obtain a good removal of waste gases. At these quantities a favourable degree of hydroxyl radical formation occurs, whereby the TOC and/or VOC can be degraded to CO₂ and H₂O.

Another aspect of the invention relates to the alkaline water which comprises a salt, wherein the anion of the salt is a hydroxide. In a specific embodiment the hydroxide is provided in a quantity of up to 1100 grams of hydroxide, preferably between 110 and 360 grams of hydroxide per gram of carbon in the air deriving from composting waste. The use of alkaline water comprising a hydroxide has the advantage that in this specific application of ozone and hydrogen peroxide a modified reaction takes place for the purpose of degrading TOC and/or VOC.

A preferred embodiment of the present invention relates to ozone that is removed after being mixed into the airflow. Excess ozone must be removed before the airflow can be introduced into the environment. Use can be made for this purpose of an ozone destructor or an active carbon filter and the like.

An aspect of the invention relates to the air which, prior to the mixing of peroxide and ozone, has a relative humidity (RH) of at least 90%, preferably more than 95% and most preferably more than 99%. At such a high air humidity the air with waste gases for treatment can be made very moist in simple and efficient manner.

A final aspect of the invention relates to the quantities of ozone, hydrogen peroxide and/or alkaline water which can be adjusted to the amount of carbon present per m³ in the air deriving from composting waste. Because the supplied air from composting waste is variable in respect of the composition, temperature, relative humidity, concentration of TOC and/or VOC, this aspect of the invention has the advantage that it is possible to anticipate the changing situations in respect of the quality and quantity of the air for treatment.

The invention is further described herein below on the basis of several examples, which are in no way intended to be limitative for the invention.

### Examples

Waste gases from an installation with a concentration of TOC of an average of 150 mg of C/Nm³ with fluctuations of 80-250 mg of C/Nm³ are analysed. About 87% of the TOC consists of VOC. The TOC and/or VOC consists for instance of methane and hydrocarbons present in the outside air. The emission of the discharge gas may comprise a maximum of 20 mg of VOC/Nm³. The emission of VOC amounts to an average of 130 mg of VOC/Nm³, with fluctuations of 65-235 mg of C/Nm³. This must be reduced to an average of 20 mg of VOC/Nm³, which results in a desired reduction of TOC of at least 85%.

In this situation the discharge gas flow rate is 40,000 m³/hour, the temperature 35°C - 48°C peak, the RH 100%, the TOC emission an average of 150 mg/m³, of which 87% VOC, the TOC load 6.0 kg/hour, of which 5.2 kg/hour of VOC, the diameter of discharge gas suction line 1,250 mm, the diameter of discharge gas pressure line 1,000 mm and a chimney height of 25 m above ground level.

Extraction takes place with two fans which are mounted on the washing space of 4.4 x 16 m with a height of 5 m. The washing space has 0.4 m of water. The free height is 4 m. A flow guide means is arranged in the washing space in the middle over a length of about 14 m. This means that the discharge gas flow travels 2 x 15 m. The diameter is 2.2 x 4 m. The two fans transport the discharge gas to the chimney. The discharge gases are carried untreated into the outside air via the chimney.

The combination of peroxide and ozone is now applied for the first time for the purpose of cleaning air which is first made very moist for this purpose by spraying water with a set pH. Peroxide and ozone are then mixed into the airflow.

The application is intended for air from, among others, Integral Biological Treatment (IBT) installations, composting installations, biological drying installations and the like.

The hydrogen peroxide is drawn from a hydrogen peroxide tank. The pH value of the circulation water is held at any value between 8.5 and 9.5. The sodium hydroxide, in principle 33% or 50%, is drawn from the sodium hydroxide tank. The sequence of addition to the discharge gas flow is to first atomize the circulation water, and then the ozone and peroxide simultaneously.

Atomizing of alkaline water takes place in the straight part of the suction pipe on top of the washing space, in two steps by means of sprayers. Ozone and hydrogen peroxide are added at the bottom of the pipe/entry to the washing space. Ozone is supplied by means of an air sprayer and hydrogen peroxide by means of atomization. The residual ozone is destroyed in an active carbon filter.

An installation according to the invention consists for instance of:
1. A packed gas washer with spray nozzle and dispensing system for ozone and atomizing of hydrogen peroxide. Sump with circulation pump.
2. Ozone generator with ozone analyser.
3. Oxygen supply from oxygen bottle.
4. Sodium hydroxide from plastic tank.
5. Hydrogen peroxide from plastic container.
6. Water supply manual.
7. Active carbon filter.
8. Fans, two units for the purpose of a suction pressure of about - 1 mbar.

In an experiment the flow rate of the discharge gases by means of the two fans amounted to an average of 300 m³/hour. The discharge gas was supplied to the gas washer. The circulation of the gas washer varied from 100 - 600 litre/hour, adjustable on the flow meter by means of a valve. The ozone dosage in the gas washer varied from 5 gr/hour to 31 gr/hour. The hydrogen peroxide dosage varied from 0.25 to 4 litre/hour. The pH value of the sump varied from 9.0 to 9.8, measured on handheld pH meter.

The oxygen supply was held constant at 7 litre/min. via a pressure reducing valve. This 7 litre/min to be divided as follows, about 2 litre/min on the ozone analyser and 2 litre/min on the ozone generator.

In another experiment ozone and the hydrogen dosage were introduced into the gas washer at the bottom. About 100 mm below the water level. Discharge gas flow rate about 300 m³/hour. The flow direction of the discharge gas from bottom to top. The ozone supply an average of 25 gr/hour. The hydrogen peroxide supply was 2.5 litre/hour on average. The washing water circulation was an average of 300 litre/hour, pH 9.2. The average result was a VOC reduction of 8%.

In a second experiment the supply of ozone and hydrogen peroxide was connected to a mixing sprayer in the liquid. Discharge gas flow rate 300 m³/hour. The flow direction of the discharge gas was from bottom to top. Ozone supply, hydrogen peroxide, circulation and pH unchanged. The average result was a VOC reduction of 50%.

The parameters were then changed stepwise each time, as was the ratio of ozone and hydrogen peroxide. pH unchanged. The reduction of 50% was not improved.

In a third experiment the following parameters applied. Discharge gas flow rate 300 m³/hour. The supply of ozone and peroxide was moved to the top of the gas washer, first above the sprayer and then below the circulation water sprayer. Water circulation at 100 litre/hour, ozone at 15 gr/hour, hydrogen peroxide at 0.3 litre/hour. pH unchanged. The average result was a VOC reduction of 88%. Best measured result was a reduction of 94%. Different settings were then implemented, the average result was not improved.

## Claims

1. Method for removing waste gases from air deriving from composting waste, comprising of first making the air very moist by spraying alkaline water into the air and subsequently mixing peroxide and ozone into the airflow.

2. Method as claimed in claim 1, wherein the air deriving from composting waste comprises per m³ between 20 mg and 10000 mg of carbon, preferably between 50 mg and 1000 mg of carbon.

3. Method as claimed in claim 1 or 2, wherein the air has a temperature between 20°C and 80°C, preferably between 40°C and 70°C, when first made very moist,

4. Method as claimed in claim 1 or 2, wherein the air has a temperature between 20°C and 80°C, preferably between 40°C and 70°C, during the spraying of alkaline water.

5. Method as claimed in claim 1 or 2, wherein the airflow has a temperature between 20°C and 80°C, preferably between 40°C and 70°C, during the mixing of peroxide and ozone.

6. Method as claimed in any of the claims 1-5, wherein the ozone is provided in a quantity of between 0.05 and 1.70 grams of ozone, preferably between 0.20 and 0.70 grams of ozone, per gram of carbon in the air deriving from composting waste.

7. Method as claimed in any of the claims 1-6, wherein the hydrogen peroxide is provided in quantity of between 0.1 and 35 grams of hydrogen peroxide of 35% by weight, preferably between 1 and 3.5 grams of hydrogen peroxide of 35% by weight, per gram of carbon in the air deriving from composting waste.

8. Method as claimed in any of the claims 1-7, wherein the alkaline water comprises a salt, wherein the anion of the salt is a hydroxide.

9. Method as claimed in claim 8, wherein the hydroxide is provided in a quantity of up to 1100 grams of hydroxide, preferably between 110 and 360 grams of hydroxide per gram of carbon in the air deriving from composting waste.

10. Method as claimed in any of the claims 1-9, wherein the ozone is removed after being mixed into the airflow.

11. Method as claimed in any of the claims 1-10, wherein the air prior to the mixing of peroxide and ozone has a relative humidity (RH) of at least 90%, preferably more than 95% and most preferably more than 99%.

12. Method as claimed in any of the claims 1-11, wherein the alkaline water has a pH of at least 8, preferably a pH of at least 9.

13. Method as claimed in any of the claims 1-12, wherein the quantities of ozone, hydrogen peroxide and/or alkaline water can be adjusted to the amount of carbon present per m³ in the air deriving from composting waste.
